# EUROPEAN PATENT APPLICATION

(11) **EP 4 403 119 A1**
(43) Date of publication of application: **24.07.2024**
(21) Application number: 22870055.5
(22) Date of filing: 16.09.2022
(51) Int. Cl.: A61B 17/122, A61B 17/128

(54) **AUXILIARY DEVICE FOR FACILITATING PLACEMENT OF THE LIGATING DEVICE INTO TARGET ORGAN**

(30) Priority: 17.09.2021 JP 2021152653
(71) Applicant: Jichi Medical University, Tokyo 102-0093 (JP); Teijin Medical Technologies Co., Ltd., Osaka 530-0005 (JP)
(72) Inventor: KANEDA, Yuji, Shimotsuke-shi, Tochigi 329-0498 (JP); HIRATA, Masumi, Osaka-shi, Osaka 530-0005 (JP); KAWABE, Yasuhiro, Osaka-shi, Osaka 530-0005 (JP); MIKAMI, Katsuhiro, Osaka-shi, Osaka 530-0005 (JP)
(74) Representative: Ipsilon
(86) International application number: PCT/JP2022/034734
(87) International publication number: WO 2023/042908

(57) **Abstract**

Ligation instrument comprising a first jaw part having a distal end and a proximal end, a second jaw part having a distal end and a proximal end and a locking feature; wherein the first jaw part and the second jaw part are configured to be rotatably connected around their respective proximal end sides; the locking feature is composed of a combination of a portion installed on the distal end side of the first jaw part and a portion installed on the distal end side of the second jaw part, the locking feature is configured to connect between the distal end side of the first jaw part and the distal end side of the second jaw part so that a target organ can be held between an inner surface of the first jaw part and an inner surface of the second jaw part, a width of a gap between the inner surface of the first jaw part and the inner surface of the second jaw part can be adjusted and a force with which the target organ is compressed can be changed by adjusting the width.

## Description

### TECHNICAL FIELD

The present invention relates to an assistive device to facilitate placement of a ligation instrument onto target organ. Specifically, the present invention relates to an assistive device to facilitate placement of ligation instrument onto target organ with an appropriate pinching force, a ligation instrument that can be placed on a target organ so as to pinches the target organ with appropriate force, and a system for ligation of target organ, comprising the assistive device and the ligation instrument.

### BACKGROUND ART

Ligation is conducted to pull and fix tissue that has been dissected due to external injury or the like; to obstruct lumen of blood vessel or fallopian tube by tying up the blood vessel or fallopian tube; to tie and secure tissue so as to close passage such as hernia orifice; or to tie up a tissue to be removed and stop the blood flow, causing necrosis and disengagement of the tissue. Proposed have been various ligation instruments for performing ligation and various assistive devices for facilitating placement of the ligation instrument on target organ.

For instance, Patent Document 1 discloses a surgical clip for pancreatectomy made of polymer, comprising a first arm part, a second arm part, and an elastic hinge part for connecting the first arm part and the second arm part, wherein the first arm part and the second arm part are connected to the elastic hinge part at each proximal end of the first arm part and the second arm part; the first arm part has a concave inner surface for clamping pancreas and a convex outer surface, the second arm part has a concave or planar inner surface for clamping the pancreas and a convex or planar outer surface; the inner surface of the first arm part and the inner surface of the second arm part face each other; and the first arm part comprises a flexible hook portion curved toward the second arm part at a distal end of the first arm part, the flexible curved hook portion is configured such that a distal end of the second arm part passes inside the flexible curved hook portion and engages the flexible curved hook portion to lock the surgical clip in closed position; the first arm part and the second arm part in opened position maintain substantially the same shape in the closed position; the inner surface of the first arm part and the inner surface of the second arm part when clamping the pancreas are configured such that a space is created between the two inner surfaces. The clip can be used with an applier. The applier comprises two leg parts in a scissor shape so that the clip can be set between the two leg parts to be opened. Each of the two leg parts has a locking mechanism inside, each locking mechanism seems to be capable of engaging a protrusion portion on the clip to secure the clip.

Patent Document 2 discloses a method for at least partially occluding a lumen, with a clamping device comprising a first pressure applying jaw part and a second pressure applying jaw part in a distal end of the clamping device and a sensor in at least one of the pressure applying jaw parts for sensing position of one or more lumens, wherein the first pressure applying jaw part and the second pressure applying jaw part have opposing luminal clip receiving surfaces, wherein the method comprises releasably securing a luminal clip between the opposing luminal clip receiving surfaces; sensing position of the lumen using the sensor; and closing the pressure applying jaw parts to compress the lumen using the luminal clip.

Patent document 4 discloses a clamping device for minimum invasive surgery, comprising: a) an elongated surface member having a proximal end and a distal end, b) a biocompatible deformable material placed on the surface member at the distal end of the surface member; and c) a flexible band having a proximal end and a distal end; the distal end of the flexible band is coupled to the distal end of the surface member; the flexible band forming a closed loop with the biocompatible deformable material on the surface member; the closed loop is fitable to a tissue or organ or a part thereof during the minimum invasive surgery; the proximal end of the flexible band is configured to be adjustably tensioned, thereby contracting or elongating a part of the flexible band that forms the closed loop with the biocompatible deformable material and the loop is thereby capable of clamping the tissue or organ or the part thereof.

Patent Document 5 discloses a laparoscopic clip applier, comprising a handle; a rigid sleeve extending from the handle; a plurality of interconnected clips and a deployment feature operable by the handle; wherein each of the clips normally opens by a proximal spring integral with said clip; the clip is housed in the sleeve; the clip comprises a self-locking feature at the distal end of the clip; each of the clips is closed and unlocked when the clip is housed in the sleeve; wherein the deployment feature is configured for advancing the clip toward the distal end of the sleeve, thereby preparing for deployment by exposing the most distal clip of the clips from the distal end of the sleeve so as to position the most distal clip and to open the most distal clip; advancing the sleeve relative to the exposed clip until the self-locking feature of the exposed clip is engaged to close the exposed clip over biological tissue, thereby placing the exposed clip; retracting the sleeve relative to the exposed clip, thereby exposing an interconnection feature disposed at the proximal end of the exposed clip, and allowing the exposed clip to be separated from the clips housed in the sleeve.

Patent Document 3 discloses a surgical instrument, comprising opposing jaws, a handle, and at least one pressure sensor. A stiffness of pancreas is measured during surgery using the surgical instrument and using of the measurement results seems to cause the surgical time to be shorten.

### PRIOR ART LIST

### PATENT DOCUMENTS

Patent document 1 : WO2020/189666A
Patent Document 2 : US2009/0287088A
Patent document 3 : US11013453B
Patent Document 4 : JP2016-531670A (WO2015/021443A)
Patent Document 5 : JP2016-529929A (WO2015/040621A)

### SUMMARY OF THE INVENTION

### PROBLEMS TO BE RESOLVED BY THE INVENTION

An object of the present invention is to provide an assistive device to facilitate placement of a ligation instrument onto a target organ with an appropriate pinching force, a ligation instrument that can be placed on a target organ so as to pinches a target organ with appropriate force, and a system for ligation of a target organ, comprising the assistive device and the ligation instrument.

### MEANS FOR SOLVING THE PROBLEMS

Intensive studies to solve the above problems result in completion of the present invention including the following embodiments.
[1] A system for ligation of target organ, wherein the system comprises
   a ligation instrument,
   a feature for observing a flow of blood to nourish a target organ, and
   an assistive device to facilitate placement of the ligation instrument onto the target organ;
   wherein the ligation instrument comprises a first jaw part having a distal end and a proximal end, a second jaw part having a distal end and a proximal end, and a locking feature; the first jaw part and the second jaw part are configured to be rotatably connected around their respective proximal end sides; the locking feature is composed of a combination of a portion installed on the distal end side of the first jaw part and a portion installed on the distal end side of the second jaw part, and the locking feature is configured to connect between the distal end side of the first jaw part and the distal end side of the second jaw part so that the target organ can be held between an inner surface of the first jaw part and an inner surface of the second jaw part, a width of a gap between the inner surface of the first jaw part and the inner surface of the second jaw part can be adjusted and a force with which the target organ is pinched can be changed by adjusting the width,
   wherein the assistive device to facilitate placement of the ligation instrument onto the target organ (hereinafter, it may be referred to as "placement assistive device"); wherein the assistive device comprises
   a shaft having a distal end and a proximal end,
   a launch pad feature on a side of the distal end of the shaft,
   a handling feature on a side of the proximal end of the shaft, and
   a transmission feature configured to connect the handling feature to the launch pad feature;
   the launch pad feature is configured to releasably load the ligation instrument thereon, and the transmission feature is configured to transfer an action/motion from the handling feature to the ligation instrument to adjust a breadth for being pinched by the first jaw part and the second jaw part so that the force with which the ligation instrument pinches the target organ can be changed.
[2] The system according to [1], further comprising a feature for observing the force with which the ligation instrument pinches the target organ.
[3] The system according to [1] or [2], wherein the ligation instrument is configured to limit the force with which the ligation instrument pinches the target organ when the ligation instrument has been placed on the target organ so that the force does not stop the flow of blood that nourishes the target organ and substantially stops a leakage of secretions from the target organ.
[4] An assistive device to facilitate placement of ligation instrument onto target organ; wherein the assistive device comprises
   a shaft having a distal end and a proximal end,
   a launch pad feature on a side of the distal end of the shaft,
   a handling feature on a side of the proximal end of the shaft, and
   a transmission feature configured to connect the handling feature to the launch pad feature;
   wherein the launch pad feature is configured to releasably load the ligation instrument thereon, and the transmission feature is configured to transfer an action/motion from the handling feature to the ligation instrument to adjust a breadth for being pinched by a first jaw part and a second jaw part comprised in the ligation instrument so that a force with which the ligation instrument pinches the target organ can be changed.
[5] An assistive device to facilitate placement of ligation instrument onto target organ; wherein the assistive device comprises
   a launch pad feature, and
   a transmission feature configured to connect a surgical support robot to the launch pad feature;
   wherein the launch pad feature is configured to releasably load the ligation instrument thereon and to be installed to the surgical support robot, and the transmission feature is configured to transfer an action/motion from the surgical support robot to the ligation instrument to adjust a breadth for being pinched by a first jaw part and a second jaw part comprised in the ligation instrument so that a force with which the ligation instrument pinches the target organ can be changed.
[6] The assistive device according to [4] or [5], wherein the ligation instrument comprises :
   the first jaw part having a distal end and a proximal end,
   the second jaw part having a distal end and a proximal end and
   a locking feature;
   wherein the first jaw part and the second jaw part are configured to be rotatably connected around their respective proximal end sides;
   wherein the locking feature is composed of a combination of a portion installed on the distal end side of the first jaw part and a portion installed on the distal end side of the second jaw part, and the locking feature is configured to connect between the distal end side of the first jaw part and the distal end side of the second jaw part so that the target organ can be held between an inner surface of the first jaw part and an inner surface of the second jaw part, a width of a gap between the inner surface of the first jaw part and the inner surface of the second jaw part can be adjusted and the force with which the target organ is pinched can be changed by adjusting the width.
[7] The assistive device according to [4], [5] or [6], wherein the ligation instrument is configured to limit the force with which the ligation instrument pinches the target organ when the ligation instrument has been placed on the target organ so that the force does not stop a flow of blood that nourishes the target organ and substantially stops a leakage of secretions from the target organ.
[8] The assistive device according to [4], [5], [6] or [7], further comprising a feature for observing the force with which the ligation instrument pinches the target organ.
[9] A ligation instrument comprising :
   a first jaw part having a distal end and a proximal end,
   a second jaw part having a distal end and a proximal end and
   a locking feature;
   wherein the first jaw part and the second jaw part are configured to be rotatably connected around their respective proximal end sides;
   wherein the locking feature is composed of a combination of a portion installed on the distal end side of the first jaw part and a portion installed on the distal end side of the second jaw part, and the locking feature is configured to connect between the distal end side of the first jaw part and the distal end side of the second jaw part so that a target organ can be held between an inner surface of the first jaw part and an inner surface of the second jaw part, a width of a gap between the inner surface of the first jaw part and the inner surface of the second jaw part can be adjusted and a force with which the target organ is pinched can be changed by adjusting the width.
[10] The ligation instrument according to [9], configured to be loaded on a launch pad feature comprised in an assistive device to facilitate placement of the ligation instrument on the target organ, wherein the launch pad feature is configured to releasably load the ligation instrument thereon.
[11] The ligation instrument according to [9] or [10], wherein the ligation instrument is configured to design a breadth with which the ligation instrument pinches the target organ when the ligation instrument has been placed on the target organ so that the breadth is maximum at a middle position biased toward the distal or proximal end from each center of the first jaw part and the second jaw part, and is minimum at an edge position of the distal or proximal end.
[12] The ligation instrument according to any one of [9] to [11], wherein the ligation instrument is configured to limit the force with which the ligation instrument pinches the target organ when the ligation instrument has been placed on the target organ so that the force does not stop a flow of blood that nourishes the target organ and substantially stops a leakage of secretions from the target organ.
[13] The ligation instrument according to any one of [9] to [12], further comprising a feature for observing the force with which the ligation instrument pinches the target organ.

### ADVANTAGEOUS EFFECTS OF THE INVENTION

The placement assistive device in the present invention facilitates placement of a ligation instrument onto a target organ with an appropriate pinching force. The ligation instrument in the present invention can be placed on a target organ so as to pinch the target organ with an appropriate force. The placement assistive device in the present invention or the system for ligation of a target organ in the present invention makes it easy to place the ligation instrument in a shorter time so as to pinch the target organ with an appropriate force. According to the present invention, secretion leakage due to insufficient tightening or tissue necrosis or cell necrosis due to excessive tightening can be effectively prevented. According to the present invention, occurrence of various complications can be reduced, for example, in partial resection of organs such as liver, pancreas or the like.

### BRIEF DESCRIPTION OF THE DRAWINGS

[FIG. 1] is an illustration showing an example of the ligation instrument (in a stress-free state) of the present invention.
[FIG. 2] is an illustration showing an example of the placement assistive device of the present invention in the state loaded with the ligation instrument of the present invention.
[FIG. 3] is an illustration showing a state in which the band body is released on the placement assistive device as shown in FIG. 2.
[FIG. 4] is an illustration showing a state in which the band body and the second jaw part are released on the placement assistive device as shown in FIG. 2.
[FIG. 5] is an illustration showing a state in which the target organ is temporarily locked with the ligation instrument on the placement assistive device as shown in FIG. 2.
[FIG. 6] is an illustration showing a state in which the ligation instrument of the present invention is released from the placement assistive device as shown in FIG. 2 and the target organ has been firmly locked with the ligation instrument.
[FIG. 7] is an illustration showing an example of a state in which the target organ is temporarily locked with the ligation instrument of the present invention.
[FIG. 8] is an illustration showing another example of the placement assistive device of the present invention in the state before loading the ligation instrument of the present invention thereon.
[FIG. 9] is an illustration showing another example of the placement assistive device of the present invention in the state loaded with the ligation instrument of the present invention thereon.
[FIG. 10] is an illustration showing an state of opening the arms of the placement assistive device as shown in FIG. 9.
[FIG. 11] is an illustration showing a state in which the band body is released on the placement assistive device as shown in FIG. 9.
[FIG. 12] is an illustration showing a state in which the target organ is inserted within the ligation instrument on the placement assistive device as shown in FIG. 9.
[FIG. 13] is an illustration showing a state in which the target organ is temporarily locked with the ligation instrument on the placement assistive device as shown in FIG. 9.
[FIG. 14] is an illustration showing a state in which the ligation instrument of the present invention is released from the placement assistive device as shown in FIG. 9 and the target organ has been firmly locked with the ligation instrument.
[FIG. 15] is an illustration showing the placement assistive device as shown in FIG. 9 in the state that the ligation instrument of the present invention has been released.

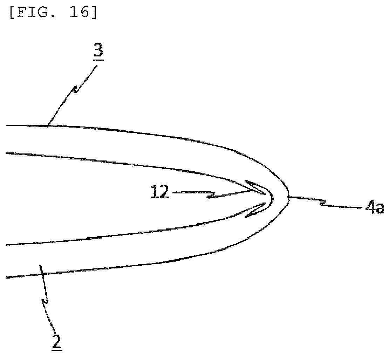

[FIG. 16] is an illustration showing an example of the connection part 4a.
[FIG. 17] is an illustration showing some examples of the internal shape of the ring, wherein the ring is formed by the first jaw part, the second jaw part and a part of the band body, when the placement is completed.

### EMBODIMENTS FOR CARRYING OUT THE INVENTION

Embodiments of the present invention will be described with reference to the drawings. The system for ligating a target organ of the present invention comprises a ligation instrument, a feature for observing a flow of blood which nourishes the target organ, and an assistive device for facilitating the placement of the ligation instrument on the target organ.

The ligation instrument of the present invention comprises a first jaw part 2, a second jaw part 3 and a locking feature.

The first jaw part 2 is a rod-shaped member having a distal end 2d and a proximal end 2p. The second jaw part 3 is a rod-shaped member having a distal end 3d and a proximal end 3p. The first jaw part and the second jaw part are preferably made of a medical material, more preferably made of a biocompatible polymer or a biocompatible metal, and even more preferably made of a biodegradable and absorbable polymer. The first jaw part and the second jaw part may be formed in bulk (mass), may be made of fibers such as nets, woven fabrics, non-woven fabrics, etc. In order to make it difficult to bend, it is preferable that it is formed in bulk.

The ligation instrument of the present invention can be obtained, for example, by integrally forming a shape consisting of parts or individually forming a shape of each part, using a polymer being a medical material, by a known resin molding method. As the polymer, mentioned can be lactic acid polymer, lactic acid-glycolic acid polymer, trimethylene carbonate polymer, dioxanone polymer, polyethylene glycol polymer, lactone polymer and the like. These polymers can be used alone or in combination of two or more. As the resin molding method, mentioned can be injection molding, compression molding, 3D printing molding and the like.

The first jaw part and the second jaw part are rotatably coupled around their respective proximal end sides as an axle. By rotation, the first jaw part and the second jaw part can be configured such that their distal ends are arranged at various angles (eg, 0 degree or more and 360 degrees or less) around the axle. Examples of the configuration can include a configuration in which both distal ends are arranged at about 180 degrees around the axle, a configuration in which both distal ends are arranged at approximately 0 degree around the axle, or the other configurations. In the configuration in which both distal ends are arranged at 0 degree to about 25 degrees around the axle, the inner surface of the first jaw part and the inner surface of the second jaw part face each other. The target organ is sandwiched between the two inner surfaces.

The part (connection part 4a), where the proximal end of the first jaw part 2 and the proximal end of the second jaw part 3 are connected, may be, for example, composed of a short flexible band member or a hinge member. Further, the connection part 4a may have a structure that allows the connection to be released, so that the first jaw part 2 and the second jaw part 3 can be separated as appropriate.

The connection part 4a may be composed of U-shaped flexible strip member, C-shaped flexible strip member, or U- or C-shaped flexible strip member provided with the peninsula portion 12 on the inside that protrudes from each jaw part toward the apex of the connection part 4a. The connection part 4a having such shape can make an interspace between the first jaw part and the second jaw part more parallel or narrower, and the target organ can be compressed uniformly, or it is possible to reduce risk that a part of the target organ is chewed by the connection part 4a. The installation of the peninsular portion can especially expect more effective in reducing the risk that a part of the target organ is chewed by the connection part 4a. The connection part 4a is preferably made of a medical material, more preferably made of a biocompatible polymer or a biocompatible metal, and even more preferably made of a biodegradable and absorbable polymer.

The inner surface of the first jaw part and the inner surface of the second jaw part may each independently be straight between the distal end and the proximal end, concave inward curve between the distal end and the proximal end, alternatively, convex inward curve between the distal end and the proximal end. Specifically, mentioned can be a configuration in which both of the inner surface of the first jaw part and the inner surface of the second jaw part are convex inward curve between the distal end and the proximal end; a configuration in which both of the inner surface of the first jaw part and the inner surface of the second jaw part are concave inward curve between the distal end and the proximal end; a configuration in which the inner surface of the first jaw part 2 is concave inward curve between the distal end and the proximal end and the inner surface of the second jaw part 3 is convex inward curve between the distal end and the proximal end, or a configuration in which the inner surface of the first jaw part 2 is convex inward curve between the distal end and the proximal end and the inner surface of the second jaw part 3 is concave inward curve between the distal end and the proximal end. From the perspective of smoothly ligating the target organ, preferable is a configuration in which both of the inner surface of the first jaw part and the inner surface of the second jaw part are concave inward curve between the distal end and the proximal end.

When the ligation instrument of the present invention has been placed on the target organ, the ligation instrument is configured so that a breadth with which the ligation instrument pinches the target organ is maximum at a middle position biased toward the distal end or the proximal end from the center of the first jaw part and the second jaw part, and is minimal at the distal end position and the proximal end position of the first jaw part and the second jaw part, from the perspective of compressing the target organ with uniform force or from the perspective of preventing the target organ from escaping toward the free ends (the distal ends of the first jaw part and second jaw part), when closing the ligation instrument. The middle position that has the maximum breadth with which the ligation instrument pinches the target organ is biased, by preferably 3 to 45%, more preferably 8 to 35%, even more preferably 10 to 30% of the length from the distal end to the proximal end of the first jaw part and the second jaw part, from the center toward the distal or proximal end of the first jaw part and the second jaw part, respectively.

The inner surface of the first jaw part and the inner surface of the second jaw part can be designed to be a rough surface, a surface having ridges or troughs extending in the longitudinal direction, a surface having ridges or troughs extending in a reticular pattern, or a surface having a plurality of convex points or concave points. On the inner surface of the first jaw part and the inner surface of the second jaw part, especially near the edges thereof, providing ridges or troughs parallel to the longitudinal direction can enhance secretion leakage prevention and hemostatic action.

The locking feature comprises a combination of a portion installed on the distal end side of the first jaw part and a portion installed on the distal end side of the second jaw part, and is configured to connect the distal end side of the first jaw part and the distal end side of the second jaw part so that a width of a gap between the first jaw part and the second jaw part for pinching the target organ can be adjusted, and is configured to change a force with which the target organ is pinched by adjusting the gap width.

As the combination in the locking feature, mentioned can be, for example, a combination of a ratchet pawl installed on the distal end side of the second jaw part and a plurality of ratchet teeth arranged along the longitudinal direction on the band body installed on the distal end side of the first jaw part, a combination of at least one recess installed in the distal end side of the second jaw part and a plurality of protrusions arranged along the longitudinal direction on the band body installed on the distal end side of the first jaw part, or a combination of at least one protrusion installed on the distal end side of the second jaw part and a plurality of recesses arranged along the longitudinal direction in the band body installed on the distal end side of the first jaw part.

Examples of the protrusions on the locking feature can include pin, hook, and the like. Examples of the recesses in the locking feature can include hole, loop, constriction, notch and the like. The plurality of protrusions or recesses provided on the band body may be a series of spherical or annular beads like a rosary linear member, a serrated edge on both sides, a ladder-shape. Preferably, the protrusion in the locking feature has a barb to prevent it from coming out of the recess into which it is inserted. The barbed protrusion may have an inverted L shape, a T shape, a cross shape, or the like. The plurality of recesses provided in the band body may be holes in a woven or mesh as long as the protrusions can be inserted therethrough. For example, Velcro fastener may be used as a combination of a hook and a loop.

A ratchet, which is an example of a locking feature, is one of features used to limit directions of operation to one direction. A tight connection is achieved by engaging the ratchet teeth 6 with the ratchet pawl 5. There are two types of ratchet pawls : retractable and non-retractable. When the ratchet teeth are engaged with the retractable ratchet pawl, the engagement can be easily released. When the ratchet teeth are engaged with the non-retractable ratchet pawl, it is difficult to release the engagement. If a non-retractable ratchet pawl and a retractable ratchet pawl are installed together, first the band body can be temporarily locked with the retractable ratchet pawl, and the engagement can be released to loosen the pinching force if the pinching force on the target organ is too tight. Then, when the pinching force is determined, the non-retractable ratchet pawl can be used to firmly lock the band body so that its position does not change.

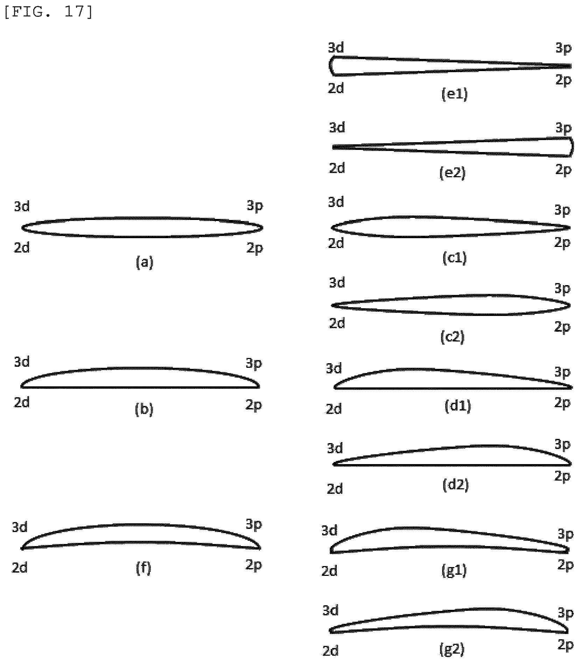

When tightened by the locking feature, the distal end of the first jaw part and the distal end of the second jaw part are connected via a portion of the band body closer to the first jaw part, the connection part 4a inflects, and a ring can be formed by the first jaw part, the second jaw part, and the portion of the band body. The ring (loop) formed when the placement is completed is not particularly limited by its shape, but it is preferable that the ring has a shape similar to a shape of the organ or organum as being compressed. The ring formed when the placement is completed may be as follows: oval or biconvex lens-shaped ring (Fig. 17(a)); semicircular or single-convex lens-shaped ring (such as a shape in which part of a circle is cut out by a straight line) (Fig. 17(b)); convex meniscus lens-shaped ring (Fig. 17(f)); triangular or trilateral ring (the first jaw part, the second jaw part, and the relevant part of the band body form three sides) (Fig. 17(e1), (e2), etc.); oval shape similar to a chicken egg; a shape in which a radius of curvature of a pointed extremity is smaller than a radius of curvature of an obtuse extremity wherein the pointed extremity and obtuse extremity are two points on circumference with which a long axis of an ellipse intersects; rings as defined by Hirotaka Hirukoi, "Short axis and oval surface of Descartes' oval line" Zugaku Kenkyu No. 68, June 1995, pp. 3-8, etc. (Fig. 17(c1), (c2) etc.); semi-elliptical or semi-oval ring (such as a shape in which part of an oval or oval shape is cut out with a straight line) (Fig. 17(d1), (d2), etc.); progressive lens-shaped rings (Fig. 17(g1), (g2), etc.); or a polygon-like ring with four or more corners or a polygon-like ring with four or more sides. It is preferable that the remaining portion of the band body is configured to follow the outer surface of the second jaw part.

A length of the portion of the band body closer to the first jaw part that connects the distal end of the first jaw part and the distal end of the second jaw part can be changed by changing the position where it is tightened. As the result, the width with which the target organ sandwiched between the inner surface of the first jaw part and the inner surface of the second jaw part can be changed. By adjusting the gap width, the force with which the target organ is pinched can be changed.

Preferably, a groove or a through hole may be provided in the end surface or outer surface of the second jaw part so that the band body can fit into the distal end of the second jaw part. Preferably, a groove may be provided on the inner surface of the first jaw part so that the distal end of the second jaw part fits into the distal end of the first jaw part. This groove or through hole prevents the distal ends of the first jaw part and the second jaw part from being misaligned.

As the mechanism for aligning the band body along the outer surface of the second jaw part 3, mentioned can be a belt loop 7 which the band body can be inserted through provided on the outer surface of the second jaw part; a groove shaped to correspond to the band body provided on the outer surface of the second jaw part; or hole, loop, hook, barbed pin, or the like provided on the outer surface of the second jaw part corresponding to barbed pin, hook, loop, hole, or the like provided on the inner surface of the band body. A ratchet pawl may be provided on the belt loop 7 for fixing the band body. A pile and hook, in which a pile (thin loop) and a hook form a pair, is fixed by the protrusion of the hook getting entangled with the pile. A hole and hook, in which a hole and a hook form a pair, is fixed by fitting the hook into the hole.

In addition, in order to fold back the band body 1 at the distal end of the second jaw part 3 and smoothly follow the outer surface of the second jaw part, the outer surface of the second jaw part preferably has a structure in which at least vicinity of the distal end is convexly curved outward. Preferably, this structure is such that when the band body 1 is fastened to the locking feature, the band body 1 is in close contact with the outer surface of the second jaw part. When the band body 1 is closely locked as described above, a stable tightening force to the target organ can be obtained and it is difficult to be loosened. Furthermore, this structure can guide a direction of the ratchet teeth on the band body 1 with respect to the ratchet pawl on the second jaw part in a direction that ensures the engagement of the ratchet tooth and the ratchet pawl. With this structure, it is possible to prevent the band body 1 from being oriented in a direction that is not appropriate for the engagement, and from applying a large load to the ratchet pawl, thereby reducing the risk of damage to the ratchet pawl.

The proximal end of the band body 1 and the distal end of the first jaw part 2 are connected via the connection part 4b. Preferably, the proximal end of the band body and the distal end of the first jaw part are connected so as to be concave inward. The connection part 4b may be, for example, a short flexible band member or a hinge member.

Furthermore, the connection part 4b may have a structure that allows the connection to be released, so that the first jaw part 2 and the band body 1 can be separated as appropriate. The connection part 4b is preferably made of a medical material, more preferably made of a biocompatible polymer or a biocompatible metal, and even more preferably made of a biodegradable and absorbable polymer.

Further, it is preferable that the band body is more flexible than the first jaw part and the second jaw part. There are no particular limitations on the method for adjusting the flexibility. For example, if the band body, the first jaw part and the second jaw part are made of the same material, the adjustment of the flexibility can be carried out by adjusting the thickness or diameter. The flexibility can be reduced by increasing the thickness of a portion of the part such as making of rib. Further, the flexibility can be increased by providing a plurality of grooves (for example, grooves between the ratchet teeth 6) on the outer surface of the band body in a direction perpendicular to the bending direction. It is preferable to provide a rib or the like on the distal end side of the band body to make rigidity higher than the proximal end side of the band body, in order to suppress buckling and facilitate insertion into the locking feature.

The locking feature is preferably made of a medical material, more preferably a biocompatible polymer or a biocompatible metal, and even more preferably a biodegradable absorbable polymer. The band body may be formed in bulk (mass) or may be made of fibers such as net, woven fabric, non-woven fabric, or the like.

In addition, the locking feature (the band body, etc.), the first jaw part and the second jaw part are not particularly limited in their length, diameter or thickness, largeness, elastic modulus, or the like. For example, they can be set as appropriate depending on shape and size of the target organ to be treated.

Preferably, the ligation instrument of the present invention is configured such that the ligation instrument can be loaded on the launch pad feature, wherein the launch pad feature comprised in the assistive device for facilitating the placement of the ligation instrument on a target organ is configured to releasably load the ligation instrument thereon. Specifically, the ligation instrument may have a receiving feature corresponding to the releasably loading feature. For example, when the launch pad feature has a chuck or the other configured to fasten the ligation instrument, the ligation instrument of the present invention has a chuck receiver that is the fastened part. Furthermore, the ligation instrument of the present invention is preferably configured to pass through a narrow passage such as a port for laparoscopic operation. The configuration of the ligation instrument preferably corresponds to the configuration of the launch pad feature. For example, if the launch pad feature has a two-pronged fork-shaped part, the ligation instrument of the present invention is preferably configured to be fitted into a gap between two teeth in the two-pronged fork. If the launch pad feature has a capsule-shaped part with a lid, the ligation instrument of the present invention is preferably configured to be fitted inside the capsule.

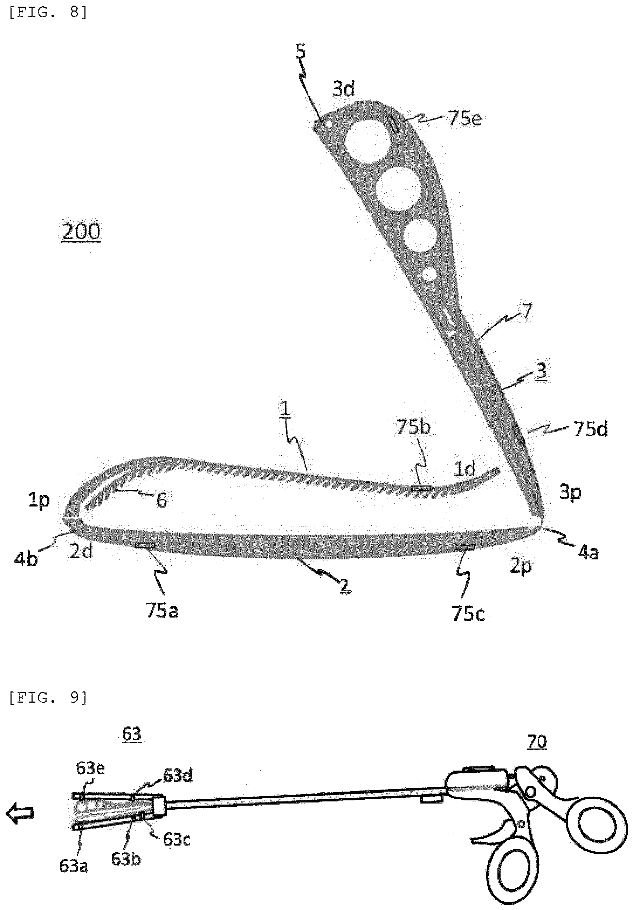

The ligation instrument of the present invention is preferably configured to arrange the band body 1 and the first jaw part 2 in a stress-free state so that the target organ 49 can be inserted into therebetween. The ligation instrument of the present invention is more preferably configured to arrange the band body 1, the first jaw part 2 and the second jaw part 3 in a stress-free state so that the target organ 49 can be inserted into thereamong. In addition, as shown in FIG. 8, when the band body 1 is folded toward the first jaw part 2, the length of the band body 1 is preferably set such that the band body 1 is housed in between the first jaw part 2 and the second jaw part 3.

The ligation instrument of the present invention may further comprise a feature for observing the force with which the ligation instrument pinches the target organ. As the feature for observing the force with which the ligation instrument pinches the target organ, mentioned can be a pressure sensor, a strain sensor, or the like. The feature for observing the pinching force can be placed at any desired position depending on principle of measurement. For example, it can be installed on the inner surface of the first jaw part or the second jaw part, on the connection part 4a of the first jaw part and the second jaw part, or on the other position. Observed values can be transmitted by the transmission feature as described below or by wireless communication. This makes it possible to know some information, for example, that the ligation instrument has come off the target organ after surgery. The ligation instrument is configured to limit the force with which the ligation instrument pinches the target organ when the ligation instrument has been placed on the target organ so that the force does not stop the flow of blood that nourishes the target organ and substantially stops the leakage of secretions from the target organ. A load limiter or the like can be used to prevent excessive compression.

In the present invention, "leakage of secretions from the target organ" means leakage of secretions from stump or wound of the target organ. Postoperative pancreatic fistula is defined by, for example, International Study Group for Pancreatic Surgery (ISGPS). The leakage of secretions is substantially stopped means, for example, the concentration of secretions (e.g., amylase in the case of pancreas, bilirubin in case of liver) contained in a drainage fluid is not more than three times the normal level in serum after the third day after surgery, or the concentration of secretions (e.g., amylase in the case of pancreas, bilirubin in case of liver) contained in ascites is not more than three times, preferably not more than two times, more preferably not more than one time the normal level in serum during the surgery.

The assistive device for facilitating the placement of the ligation instrument of the present invention on a target organ (placement assistive device) comprises a shaft, a launch pad feature, a handling feature, and a transmission feature.

The shaft is a rod-like member having a distal end and a proximal end. A port for laparoscopic operation (hereinafter called laparoport) may be provided in abdominal wall, wherein for example, in laparoscopic operation, some surgical instruments may be inserted into abdominal cavity from outside of the body through the laparoport. A diameter and length of the shaft are not particularly limited as long as it does not interfere with handling during the operation. For example, in laparoscopic operation, the shaft can have a diameter enough to pass through the laparoport and a length enough to reach the target organ. The shaft may have a lumen communicating from the proximal end to the distal end. A transmission feature, etc., which will be described later, can be housed in the lumen.

In addition, the surgical procedure may be performed by a doctor directly operating the ligation instrument, the assistive device or the system for ligating target organ of the present invention, or a doctor indirectly operating the ligation instrument, the assistive device or the system for ligating target organ of the present invention via a surgical support robot. The surgical support robot comprises, for example, a patient section equipped with a robot arm configured to perform surgical procedures on a patient, and a surgeon section configured to allow a doctor to control movements of the patient section. As the surgical support robot, mentioned can be hinotori surgical robot system (manufactured by Medicaroid), da Vinci Surgical System (manufactured by Intuitive Surgical), EMARO surgical robot system (manufactured by Riverfield), Mazor X robot system (manufactured by Medtronic Japan) or the like.

The launch pad feature is provided on the side of the distal end of the shaft. The handling feature is provided on the side of the proximal end of the shaft. The transmission feature is configured to connect the handling feature to the launch pad feature. The transmission feature is configured to transfer an action/motion from the handling feature to the launch pad feature or the ligation instrument so as to adjust a breadth with which the target organ is pinched by the first jaw part and the second jaw part so that the force with which the ligation instrument pinches the target organ can be changed.

The transmission feature can comprises a feature for transmitting mechanical driving force, a feature for transmitting electrical/electronic signals, or the like. The handling feature can comprises one or more of the followings : a grasped part to be grasped by the operator, an operation part, such as a button, a knob, a dial, a lever, a touch panel or the like, to be operated by the operator, a connection part to be connected to external equipment such as a monitor or a measuring instrument or the like. The handling feature may comprise a portion for displaying information regarding the blood flow that nourishes the target organ, the force with which the ligation instrument pinches the target organ or the like. The portion for displaying such information may be installed in a place where the operator can easily see it, for example, a place where images taken with a laparoscope are displayed.

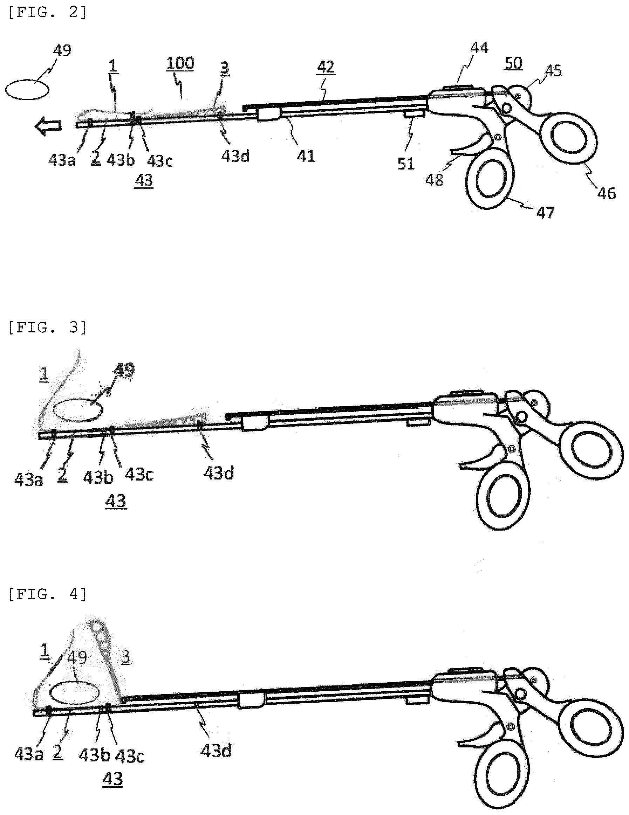

The launch pad feature is configured to releasably load the ligation instrument to be placed on target organ thereon. Examples of the releasably loading feature can include chuck, forceps, or the like as being configured to grasp the ligation instrument. The ligation instrument loaded on the launch pad feature can be configured to pass through the laparoport, for example, the distal end of the first jaw part and the distal end of the second jaw part may be arranged at approximately 180 degrees, or the distal end of the first jaw part and the distal end of the second jaw part may be arranged at approximately 0 degree. Then, the loading feature in the launch pad feature is activated via the transmission feature by operating the handling feature, so as to arrange the first jaw part and second jaw part of the ligation instrument loaded on the launch pad feature so that the target organ can be inserted into therebetween (Figures 3-4, Figures 10-11, etc.).

In robotic surgery, the handling feature constituting the assistive device of the present invention may be operated with forceps or the like attached to the robot arm provided in the patient section of the surgery support robot. Further, if the launch pad feature is configured to be attached to the robot arm of the surgery support robot, the launch pad feature can be attached to the robot arm of the surgery support robot. When the launch pad feature is attached to the robot arm of the surgical support robot, the aforementioned shaft is equivalent to the robot arm of the surgical support robot, and the handling feature is equivalent to a control rod in the surgeon section of the surgical support robot. And the transmission feature is configured to transmit an action/motion from the surgical support robot to the launch pad feature or the ligation instrument, to adjust the breadth with which the target organ is pinched between the first jaw part and second jaw part and the force with which the ligation instrument pinches the target organ can be changed.

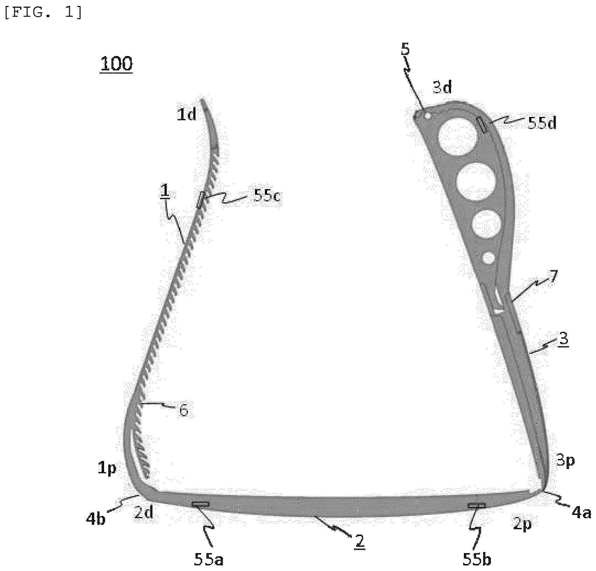

In one embodiment, for example, in a stress-free state, the ligation instrument 100 is configured to have arrangement in such a way that the band body 1, the first jaw part 2 and the second jaw part 3 have capable of enclosing the target organ 49 as shown in Fig. 1. The releasably loading feature such as a chuck applies stress to the ligation instrument 100, then the ligation instrument, as shown in Figure 2, is made into the following form : the distal end of the first jaw part and the distal end of the second jaw part are spread out so that they are arranged at about 180 degrees, and the band body 1 is folded so as to approach the first jaw part. The ligation instrument 100 in this form is pinched with the chucks 43a, 43b, 43c and 43d and loaded on the launch pad feature 43.

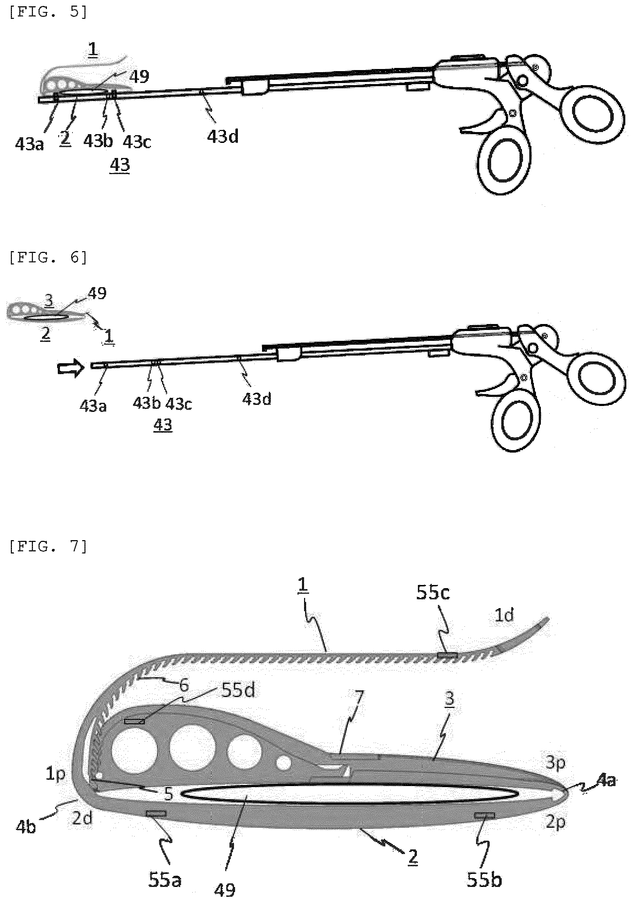

After inserting it in the abdominal cavity, the band body 1 is expanded as shown in FIG. 3 by loosening the chuck 43b that has gripped the band body 1, the second jaw part 3 is arranged as shown in FIG. 4 by loosening the chuck 43d that had gripped the second jaw part 3. The target organ 49 is inserted into between the first jaw part and the second jaw part, the second jaw part 3 is pushed down with the rod 42 being one of the transmission feature. The ratchet claw or the like at the distal end of the second jaw part is engaged with a ratchet tooth or the like on the band body 1 to temporarily lock them as shown in FIG. 5. The ligation instrument used here may be configured so that the ratchet teeth 6 provided on the band body 1 and the ratchet claw 5 provided on the second jaw part 3 can engage with each other by only the pressing force of the rod 42, and the temporary lock can be established. Specifically, for that, tailored is a length of the first jaw part 2 or the second jaw part 3, mutual position of the band body 1, the first jaw part 2 and the second jaw part 3 via the connection parts 4a and 4b (in particular, the angle formed by the band body 1 and the first jaw part 2 in a stress-free state), flexibility of the band body 1, location of the ratchet pawl 5 on the second jaw part 3, or the like.

Then, the distal end of the band body 1 is pinched with the rod 42, and pulled toward the handling feature side along the outer surface of the second jaw part. The target organ is compressed while adjusting the breadth with which the first jaw part and the second jaw part pinch the target organ. The compressing force can be converted from the pulling force of the rod 42. A level of the force can be indicated on an indicator 44 in the handling feature. The feature for observing the force that compresses the target organ is not limited to those described above, the known load measuring instrument such as a pressure sensor, a strain sensor, a spring balance or the like may be employed. When the pinching force reaches the desired level, the ligation instrument is released by loosening the chucks 43a, 43c. The ligation instrument can be coordinated to limit the force with which the ligation instrument pinches the target organ when the ligation instrument has been placed on the target organ so that the force does not stop the flow of blood that nourishes the target organ and substantially stops the leakage of secretions from the target organ based on the observed value of the flow of blood that nourishes the target organ. The ligation instrument may have been configured to limit the force with which the ligation instrument pinches the target organ when the ligation instrument has been placed on the target organ so that the force does not stop the flow of blood that nourishes the target organ and substantially stops the leakage of secretions from the target organ. Additionally, a load limiter or the like can be used to prevent excessive compression. In this embodiment, each chuck can be operated using the operation button 51, the movement of the rod 42 can be controlled using the operation dial 45, levers 46, 48, or the like.

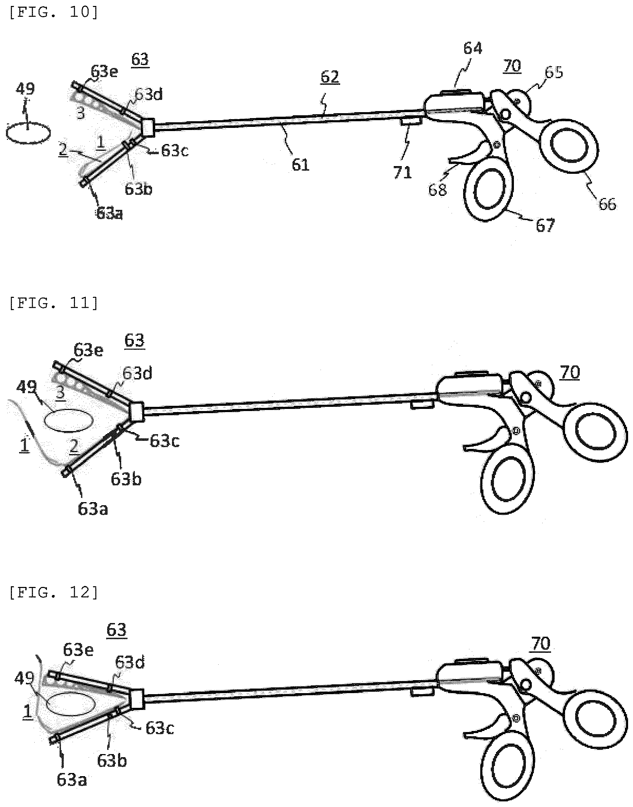

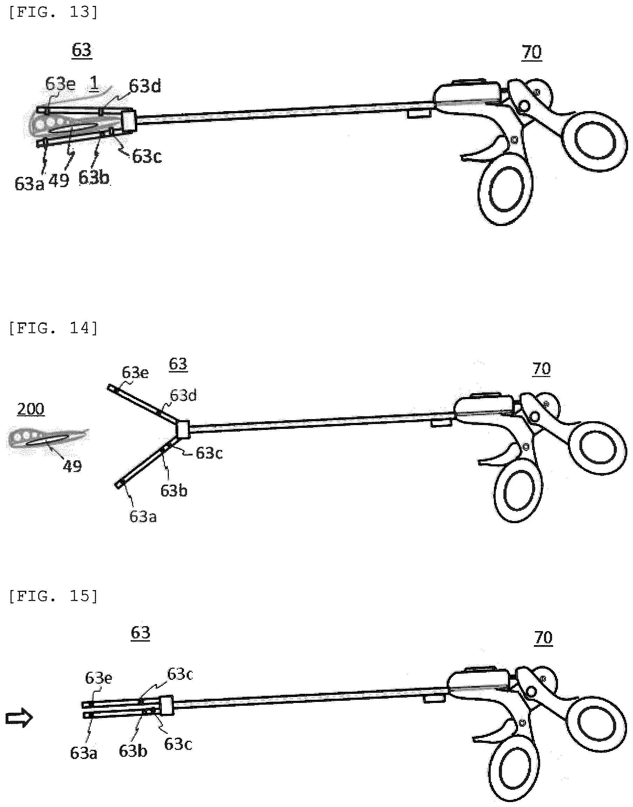

In another embodiment, the band body is interfolded between the first jaw part and the second jaw part as shown in FIG. 8, and the ligation instrument is fastened with releasably loading feature such as chucks 63a, 63b, 63c, 63d and 63e on the arms provided in the launch pad feature 63 to be loaded on the launch pad feature 63. When it is used in laparoscopic surgery, as shown in FIG. 9, the arms of the launch pad feature 63 are closed so that the distal end of the first jaw part and the distal end of the second jaw part are aligned at approximately 0 degrees, which allows to be passed through Raparoport. After it is inserted into the abdominal cavity, the arms are opened and the chuck 63b that has fastened the band body 1 is unclenched, resulting in expansion of the band body 1 as shown in FIG. 11. The target organ 49 is enclosed in between the first jaw part and the second jaw part. The arms are closed, and a ratchet claw or the like at the distal end of the second jaw part is engaged with a ratchet tooth or the like on the band body 1 to do temporarily lock as shown in FIG. 13. The ligation instrument used here also has a structure in which a temporary lock can be established only by operation via the launch pad feature 63, similar to the previous one described using FIG. 5.

As shown in FIG. 13, the band body 1 runs along the outer surface of the arm that fastens the second jaw part. The chucks 63a, 63c, 63d and 63e are eased. The distal end of the band body 1 is pinched with the distal ends of the two arms and is pulled toward the handling feature along the outer surface of the second jaw part. As the result, the target organ is pinched with a desired force by adjusting the width of the gap between the first jaw part and the second jaw part.

The feature for observing the flow of blood that nourishes the target organ can comprise feature for measuring pressure pulse waves, feature for detecting Korotkov's sound, feature for measuring light transmission, light reflection or the like, feature for measuring ultrasonic wave transmission, ultrasonic wave reflection or the like, feature for photographing with injection of a contrast agent such as fluorescent dye, or the others. The feature for observing the blood flow can further comprise feature for converting these measurement results into images. The feature for observing the blood flow may be a device solely for observing fluid flow, or may be a multifunction medical device with feature for observing fluid flow as well as the other medical functions, as long as the flow of blood that nourishes the target organ can be observed. For example, it may be installed in the aforementioned ligation instrument, the assistive device for facilitating placement on the target organ, or other medical equipments (for example, forceps, laparoport, laparoscope, etc.).

The system of the present invention can comprise, in addition to the feature for observing the flow of blood that nourishes the target organ, a feature for monitoring other conditions of the patient during surgery. As a patient monitoring feature during surgery, mentioned can be a pulse oxymeter that can measure blood oxygen concentration or the like, a capnometer that can measure carbon dioxide concentration in indrawn breath and exhaled breath, a ventilation monitor that can measure airway pressure or the like, an electrocardiographic monitor, a blood pressure monitor, a body temperature monitor, a neuromuscular monitor, and the like.

The target organ to which the present invention can be applied is not limited as long as ligation treatment is effective. As the target organ, mentioned can be, for instance, organum with a luminal structure such as blood vessel, lymphatic vessel, thoracic duct, bile duct, fallopian tube, vagina, ureter, urethra, vas deferens, trachea, or bronchi; pancreas, liver, gallbladder, spleen, kidney, bladder, uterus, ovary, testicles, lungs, heart, thyroid, esophagus, stomach, duodenum, small intestine, large intestine, lymph nodes; or the like.

The present invention can be preferably used for ligating pancreas, and more preferably for ligating a body or tail of the pancreas during pancreatic resection. The present invention can be preferably used for liver ligation, can be more preferably used for ligating liver parenchyma during hepatic hemorrhage, and can be used for the purpose of reducing hepatic hemorrhage during hepatectomy. The present invention can be preferably used for splenic ligation, can be preferably used for ligating the splenic parenchyma during splenic hemorrhage, and can be used for the purpose of reducing splenic hemorrhage during splenectomy. The present invention can be preferably used for ligating kidney, can be preferably used for ligation of renal parenchyma during kidney hemorrhage, and can be used for the purpose of reducing renal bleeding during nephrectomy.

Because the target organ is easily deformed and fragile, conventional ligation instruments used for ligating a stump of the target organ, in order to minimize tissue damage caused by ligation, such as tissue necrosis due to obstruction of blood flow within the tissue, usually have a shape that approximates the shape of the cutting surface so that pressure can be applied as evenly as possible onto a circumference of the organ stump. For example, in the case of pancreatic stump treatment, approximately circular ligation instruments have been conventionally used. By the way, if the target organ has a luminal structure, cutting of the target organ exposes an opening of the luminal structure on the cutting surface. Preventing leakage of secretion during ligation of the target organ is also one of issues, if there is the secretion, which may damage the surrounding tissues, such as digestive fluid, in the luminal structure. Examples of the issues can include pancreatic fistula. However, in conventional substantially circular ligation instruments, if the ligation is done loosely to avoid excessive pressure on the target organ, it is not possible to effectively close the opening of the lumen structure at the cutting surface of the target organ. On the other hand, when emphasis is placed on occlusion of the luminal structure, excessive pressure will be applied to the target organ, and problems of blood flow obstruction within the target organ and tissue necrosis due to the blood flow obstruction will become more serious. That is to say, there is a trade-off between the need to reduce tissue damage caused by ligation and the need to suppress fluid leakage from the opening of the lumen structure at the cutting surface of the target organ.

As mentioned above, in the present invention, by installing the first jaw part and the second jaw part having the specific shape and the specific flexibility as well as the adjustable locking feature to maintain an appropriate distance between the distal end of the first jaw part and the distal end of the second jaw part during ligation, it can be adjusted so as to minimize the obstruction of blood flow within the target organ due to pinching pressure and the resulting necrosis. And, according to the present invention, even if there is a luminal structure within the target organ, the opening of the luminal structure can be effectively occluded. That is, the present invention makes it possible both to reduce tissue damage caused by ligation and to suppress fluid leakage from the opening of the lumen structure at the cutting surface of the target organ. Also, taking the case where the target organ is the pancreas, by adjusting the force for ligating the pancreas, since the ligation instrument of the present invention can be fixed on the pancreas while maintaining an aperture of the luminal structure, the gastrointestinal tract or tissues can be sewn in pancreatico-gastrointestinal anastomosis. The present invention is preferably applied to laparoscopic surgery.

### DESCRIPTION OF CODES

1 : Band body
2 : First jaw part
3 : Second jaw part
4a : Connection part between the first jaw part and the second jaw part
4b : Connection part between the band body and the first jaw part
5 : Ratchet claw
6 : Ratchet teeth
7 : Belt loop
12 : Peninsula part
1p : Proximal end of the band body
1d : Distal end of the band body
2p : Proximal end of the first jaw part
2d : Distal end of the first jaw part
3p : Proximal end of the second jaw part
3d : Distal end of the second jaw part
41, 61 : Shaft
42, 62 : Rod
43, 63 : Launch pad feature
43a, 43b, 43c, 43d : Chuck
63a, 63b, 63c, 63d, 63e : Chuck
55a, 55b, 55c, 55d : Chuck receiver
75a, 75b, 75c, 75d, 75e : Chuck receiver
44, 64 : Part that displays information
45, 65 : Operation dial
46, 66 : Operation lever
47, 67 : Grip lever
48, 68 : Operation knob
49 : Target organ
50, 70 : Handling feature
51, 71 : Operation button
100, 200 : Ligation instrument

## Claims

1. A system for ligation of target organ,
wherein the system comprises
a ligation instrument,
a feature for observing a flow of blood to nourish a target organ, and
an assistive device to facilitate placement of the ligation instrument onto the target organ,
wherein the ligation instrument comprises a first jaw part having a distal end and a proximal end, a second jaw part having a distal end and a proximal end and a locking feature; the first jaw part and the second jaw part are configured to be rotatably connected around their respective proximal end sides; the locking feature is composed of a combination of a portion installed on the distal end side of the first jaw part and a portion installed on the distal end side of the second jaw part, and the locking feature is configured to connect between the distal end side of the first jaw part and the distal end side of the second jaw part so that the target organ can be held between an inner surface of the first jaw part and an inner surface of the second jaw part, a width of a gap between the inner surface of the first jaw part and the inner surface of the second jaw part can be adjusted and a force with which the target organ is pinched can be changed by adjusting the width,
wherein the assistive device to facilitate placement of the ligation instrument onto the target organ;
wherein the assistive device comprises
a shaft having a distal end and a proximal end,
a launch pad feature on a side of the distal end of the shaft,
a handling feature on a side of the proximal end of the shaft, and
a transmission feature configured to connect the handling feature to the launch pad feature, wherein the launch pad feature is configured to releasably load the ligation instrument thereon, and the transmission feature is configured to transfer an action/motion from the handling feature to the ligation instrument to adjust a breadth for being pinched by the first jaw part and the second jaw part so that the force with which the ligation instrument pinches the target organ can be changed.

2. The system according to claim 1, further comprising a feature for observing the force with which the ligation instrument pinches the target organ.

3. The system according to claim 1 or 2, wherein the ligation instrument is configured to limit the force with which the ligation instrument pinches the target organ when the ligation instrument has been placed on the target organ so that the force does not stop the flow of blood that nourishes the target organ and substantially stops a leakage of secretions from the target organ.

4. An assistive device to facilitate placement of ligation instrument onto target organ; wherein the assistive device comprises
a shaft having a distal end and a proximal end,
a launch pad feature on a side of the distal end of the shaft,
a handling feature on a side of the proximal end of the shaft, and
a transmission feature configured to connect the handling feature to the launch pad feature;
wherein the launch pad feature is configured to releasably load the ligation instrument thereon, and the transmission feature is configured to transfer an action/motion from the handling feature to the ligation instrument to adjust a breadth for being pinched by a first jaw part and a second jaw part comprised in the ligation instrument so that a force with which the ligation instrument pinches the target organ can be changed.

5. An assistive device to facilitate placement of ligation instrument onto target organ; wherein the assistive device comprises
a launch pad feature, and
a transmission feature configured to connect a surgical support robot to the launch pad feature;
wherein the launch pad feature is configured to releasably load the ligation instrument thereon and to be installed to the surgical support robot, and the transmission feature is configured to transfer an action/motion from the surgical support robot to the ligation instrument to adjust a breadth for being pinched by a first jaw part and a second jaw part comprised in the ligation instrument so that a force with which the ligation instrument pinches the target organ can be changed.

6. The assistive device according to claim 4 or 5, wherein the ligation instrument comprises :
the first jaw part having a distal end and a proximal end,
the second jaw part having a distal end and a proximal end and
a locking feature;
wherein the first jaw part and the second jaw part are configured to be rotatably connected around their respective proximal end sides;
wherein the locking feature is composed of a combination of a portion installed on the distal end side of the first jaw part and a portion installed on the distal end side of the second jaw part, and
the locking feature is configured to connect between the distal end side of the first jaw part and the distal end side of the second jaw part so that the target organ can be held between an inner surface of the first jaw part and an inner surface of the second jaw part, a width of a gap between the inner surface of the first jaw part and the inner surface of the second jaw part can be adjusted and the force with which the target organ is compressed can be changed by adjusting the width.

7. The assistive device according to claim 4 or 5, wherein the ligation instrument is configured to limit the force with which the ligation instrument pinches the target organ when the ligation instrument has been placed on the target organ so that the force does not stop a flow of blood that nourishes the target organ and substantially stops a leakage of secretions from the target organ.

8. The assistive device according to claim 4 or 5, further comprising a feature for observing the force with which the ligation instrument pinches the target organ.

9. A ligation instrument comprising :
a first jaw part having a distal end and a proximal end,
a second jaw part having a distal end and a proximal end and
a locking feature;
wherein the first jaw part and the second jaw part are configured to be rotatably connected around their respective proximal end sides;
wherein the locking feature is composed of a combination of a portion installed on the distal end side of the first jaw part and a portion installed on the distal end side of the second jaw part, and
the locking feature is configured to connect between the distal end side of the first jaw part and the distal end side of the second jaw part so that a target organ can be held between an inner surface of the first jaw part and an inner surface of the second jaw part, a width of a gap between the inner surface of the first jaw part and the inner surface of the second jaw part can be adjusted and a force with which the target organ is pinched can be changed by adjusting the width.

10. The ligation instrument according to claim 9, configured to be loaded on a launch pad feature comprised in an assistive device to facilitate placement of the ligation instrument on the target organ, wherein the launch pad feature is configured to releasably load the ligation instrument thereon.

11. The ligation instrument according to claim 9 or 10, wherein the ligation instrument is configured to design a breadth with which the ligation instrument pinches the target organ when the ligation instrument has been placed on the target organ so that the breadth is maximum at a middle position biased toward the distal or proximal end from each center of the first jaw part and the second jaw part, and is minimum at an edge position of the distal or proximal end.

12. The ligation instrument according to claim 9 or 10, wherein the ligation instrument is configured to limit the force with which the ligation instrument pinches the target organ when the ligation instrument has been placed on the target organ so that the force does not stop a flow of blood that nourishes the target organ and substantially stops a leakage of secretions from the target organ.

13. The ligation instrument according to claim 9 or 10, further comprising a feature for observing the force with which the ligation instrument pinches the target organ.
